# EUROPÄISCHE PATENTSCHRIFT

(11) **EP 1 597 232 B1**
(45) Veröffentlichungstag und Bekanntmachung des Hinweises auf die Patenterteilung: **13.09.2006**
(21) Anmeldenummer: 04705809.4
(22) Anmeldetag: 28.01.2004
(51) Int. Cl.: C07D 213/82, A01N 43/40

(54) **NEUE KRISTALLINE MODIFIKATION DES ANHYDRATES VON BOSCALID**
NOVEL CRYSTALLINE MODIFICATION OF THE ANHYDRATE OF BOSCALID
NOUVELLE MODIFICATION CRISTALLINE DE L'ANHYDRE DU BOSCALID

(30) Priorität: 14.02.2003 DE 10307751
(43) Veröffentlichungstag der Anmeldung: 23.11.2005
(73) Patentinhaber: BASF Aktiengesellschaft, 67056 Ludwigshafen (DE)
(72) Erfinder: MAYER, Winfried, 55270 Bubenheim (DE); ZIEGLER, Hans, 67112 Mutterstadt (DE); SCHNEIDER, Karl-Heinrich, 67271 Kleinkarlbach (DE); KRÖHL, Thomas, 55129 Mainz (DE); MAYER, Horst, 67069 Ludwigshafen (DE); ERK, Peter, 67227 Frankenthal (DE); COX, Gerhard, 67098 Bad Dürkheim (DE); STIERL, Reinhard, 67251 Freinsheim (DE)
(86) Internationale Anmeldenummer: PCT/EP2004/000703
(87) Internationale Veröffentlichungsnummer: WO 2004/072039

(56) Entgegenhaltungen:
- EP-A- 0 545 099
- WO-A-99/31979
- WO-A-03/029219
- WO-A-03/090538

## Beschreibung

Gegenstand der vorliegenden Erfindung ist monoklines, bei 147 - 148 °C schmelzendes 2-Chlor-N-(4'-chlorbiphenyl-2-yl)-nicotinamid der Formel I

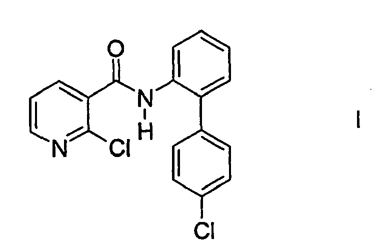

Die vorliegende Erfindung umfasst weiterhin deren Herstellung:

Monoklines, bei 144 - 145 °C schmelzendes 2-Chlor-N-(4'-chlorbiphenyl-2-yl)-nicotinamid der Formel I wird in der EP-A-545 099 und der WO 03/029219 beschrieben und ist unter dem Common Name Boscalid bekannt. In der WO 03/029219 wird das Anhydrat sowie das Hydrat des Bocalid beschrieben als auch die Herstellung des Boscalid Hydrats aus dem Boscalid Anhydrat.

Das bekannte Anhydrat des Boscalids wird in dieser Anmeldung als Modifikation I bezeichnet, das Anhydrat des Boscalids gemäß Anspruch 1 wird als Modifikation II bezeichnet.

Um aus dem Anhydrat des Boscalids ein wässriges Suspensionskonzentrat (SC) oder eine Suspoemulsion (SE) herzustellen, muss gemäß WO 03/029219 zunächst das Hydrat des Boscalids hergestellt werden, das dann in Gegenwart von Wasser und weiteren Hilfsstoffen möglichst fein vermahlen wird. Dies ist mit dem Anhydrat des Boscalids nicht möglich, da dieses beim Vermahlen mit den Hilfsstoffen in Wasser einen lehmartigen Feststoff bildet, der einen weiteren Mahlvorgang verhindert.

Gemäß WO 03/029219 wird das Hydrat des Boscalids der Modifikation I hergestellt, indem das Anhydrat des Boscalids der Modifikation I in einem wasserlöslichen Lösungsmittel gelöst wird und dann das Hydrat durch Zugabe von Wasser ausgefällt wird.

Feststoffformulierungen, wie sprühgetrocknete oder extrudierte wasserdispergierbare Granulate, lassen sich direkt aus dem Anhydrat des Boscalids der Modifikation I herstellen, ohne dass vorher eine Umwandlung zum Hydrat erfolgen muss.

Verdünnt man nun diese wasserdispergierbaren Granulate mit Wasser und mischt diese mit lösungsmittelhaltigen Pflanzenschutzpräparaten, wie zum Beispiel Emulsionskonzentraten (EC), so kann es zu Problemen bei der Ausbringung kommen, da sich Boscalidkristalle bilden, die dann die Filter in Applikationsgeräten verstopfen können.

Aufgabe der vorliegenden Erfindung war es, den bei den wasserdispergierbaren Granulaten geschilderten Mangel zu beheben.

Die Aufgabe wurde gelöst durch die Bereitstellung des monoklinen, bei 147 -148 °C schmelzenden Anhydrats des Boscalids der Formel I der Modifikation II. Überraschenderweise wurde gefunden, dass das Anhydrat des Boscalids der Formel I in Form der Modifikation II in Gegenwart von Lösungsmitteln kaum Kristallwachstum zeigt.

Die Herstellung des Anhydrates des Boscalids der Formel I der Modifikation I ist in der EP-A-545 099 und WO 03/029219 beschrieben.

Gegenstand der vorliegenden Erfindung sind weiterhin Verfahren zur Herstellung des Anhydrats des Boscalids der Modifikation II.

In einer Ausführungsform (Verfahren 1) umfasst das Verfahren die folgenden Schritte:
a) Lösen des Anhydrats der Verbindung der Formel I der Modifikation I in einem polaren organischen Lösungsmittel oder einem aromatischen Kohlenwasserstoff,
b) Präzipitation des Anhydrats der Verbindung der Formel I der Modifikation II durch Abkühlen des Lösungsmittels.

Geeignete polare Lösungsmittel sind Alkohole, Glykole, Ketone, Ether, Ester, Amide oder Gemische dieser Lösungsmittel. Weiterhin sind aromatische Kohlenwasserstoffe geeignet.

Beispiele für Alkohole sind Methanol, Ethanol oder Propanol. Besonders bevorzugt ist Methanol.

Geeignete Glykole sind beispielsweise Ethylenglykol und Dithylenglykol.

Geeignete Ketone sind beispielsweise Aceton und Cyclohexanon.

Geeignete Ether sind beispielweise Dioxan und Tetrahydrofuran.

Ein geeigneter Ester ist beispielsweise Essigester.

Ein geeignetes Amid ist beispielsweise Dimethylformamid.

Geeignete aromatische Lösungsmittel sind beispielsweise Benzol, Toluol, oder Xylol.

Das Lösen des Anhydrats der Verbindung der Formel I der Modifikation I in der Stufe a) erfolgt bei Temperaturen von 20 bis 150 °C, bevorzugt 40 bis 115 °C und besonders bevorzugt 50 bis 95 °C.

Die Präzipitation des Anhydrates der Verbindung der Formel I der Modifikation II in der Stufe b) erfolgt durch Abkühlen der in der Stufe a) erhaltenen Lösung auf Temperaturen von 0 bis 30 °C, bevorzugt 10 bis 25 °C, besonders bevorzugt 20 bis 25 °C. Die Präzipitation erfolgt über einen Zeitraum von 1 bis 24 Stunden, bevorzugt 2 bis 20 Stunden.

Besonders vorteilhaft ist die Zugabe von Impfkristallen des Anhydrats der Verbindung der Formel I der Modifikation II in der Stufe b), was die Präzipitation deutlich beschleunigt.

In einer weiteren Ausführungsform (Verfahren 2) umfasst das Verfahren die folgenden Schritte:
a) Erhitzen des Anhydrates der Verbindung der Formel I der Modifikation I auf über 150 °C bis alles geschmolzen ist,
b) Abkühlen der Schmelze unter Zugabe von Impfkristallen des Anhydrates der Verbindung der Formel I der Modifikation II.

Die Impfkristalle werden in der Stufe b) in einer Menge von 0,01 bis 20 Gew.-%, bevorzugt 0,05 bis 5 Gew.-% zugegeben.

Dieses Verfahren wird bevorzugt in einem geeigneten Edelstahlgefäß durchgeführt. Die Umwandlung des Anhydrates der Verbindung der Formel I der Modifikation I in die Modifikation II erfolgt quantitativ.

Die physikalischen Eigenschaften der beiden Modifikationen der Anhydrate der Verbindung der Formel I sind in der Tabelle 1 gegenübergestellt

**Tabelle 1**

| **Eigenschaften** | **Anhydrat, Modifikation I** | **Anhydrat, Modifikation II** |
|---|---|---|
| Molekulargewicht [g/mol] | 342 | 342 |
| Schmelzpunkt [°C] (DSC) | 144,8 | 147,2 |
| Schmelzwärme [J/g] (DSC) | 85 | 106 |
| Dichte [g/cm³] | 1,399 | 1,457 |
| Charakteristische IR-Banden [cm⁻¹] | 924, 1310, 1650 | 868, 917, 1675 |

Die Zellparameter aus den kristallographischen Untersuchungen mit einem Einkristalldiffraktometer der Fa. Siemens sind in der Tabelle 2 aufgeführt:

**Tabelle 2**

| **Parameter** | **Anhydrat, Modifikation I** | **Anhydrat, Modifikation II** |
|---|---|---|
| Klasse | Monoclinic | Monoclinic |
| Raumgruppe | P21/c | P21/c |
| a | 1479.2(3) pm | 1162.5(6) pm |
| b | 1157.67(19) pm | 1134.2(4) pm |
| c | 1872.1 (3) pm | 1283.2(5) pm |
| α | 90° | 90° |
| β | 91.993 (17)° | 114.52(4)° |
| γ | 90° | 90° |
| Volumen | 3.2038(9) nm³ | 1.5390 nm³ |
| Z | 8 | 4 |
| Dichte (berechnet) | 1.423 Mg/m³ | 1.481 Mg/m³ |
| R¹, wR² | 0.1036; 0.1699 | 0.0489; 0.1264 |

Dabei haben die angegebenen Parameter die folgende Bedeutung:
a,b,c = Kantenlängen der Elementarzelle
α,β,γ = entsprechende Winkel
Z = Anzahl der Moleküle in der Elementarzelle

### Beispiel 1:

### Herstellung des Anhydrates der Verbindung der Formel I der Modifikation II:

30 g Methanol werden in einem Schlifferlenmeyerkolben vorgelegt. Anschließend gibt man 5 g Anhydrat der Verbindung der Formel I der Modifikation I hinzu und erwärmt die Mischung im Wasserbad unter Rühren auf 55 °C bis sich alles gelöst hat (ca. 10 min). Danach wird der Kolben aus dem Heizbad genommen und anschließend über einen Zeitraum von 18 Stunden bei Umgebungstemperatur (ca. 20 °C) abkühlen lassen. Dabei kristallisiert Anhydrat die Verbindung der Formel I der Modifikation II aus. Smp 147,2 °C

### Beispiel 2:

200 g Anhydrat der Verbindung der Formel I der Modifikation I werden in einem Edelstahlgefäß auf 160°C erhitzt. Anschließend wird die Schmelze unter Rühren abgekühlt. Bei 150°C wird mit Kristallen des Anhydrats der Verbindung der Formel I der Modifikation II angeimpft und weiter abgekühlt. Dabei erhält man Anhydrat der Verbindung der Formel I der Modifikation II.

### Beispiel 3:

Herstellung eines wasserdispergierbaren Granulates aus Anhydrat der Verbindung der Formel I der Modifikation I oder II:

### Zusammensetzung:

| **Bestandteil** | **Konzentration** |
|---|---|
| Boscalid (Wirkstoff) | 30 - 60% w/w |
| Dispergiermittel (Ligninsulfonat-Salz) | 10 - 20% w/w |
| Netzmittel (Naphthalinsulfonsäure-Kondensationsprodukt-Salz) | 5 - 10% w/w |
| Antischaummittel (Silikonöl) | 0,5 - 1 % w/w |
| Stellmittel (Na-Sulfat) | ad 100 % |

### Herstellungsverfahren:

Eine Suspension geeigneter Konzentration wird angesetzt und mittels einer Perlmühle auf die gewünschte Partikelgröße vermahlen (50% < 2µm). Anschließend wird die Suspension in einem Sprühturm getrocknet.

### Vergleich der Tankmischverträglichkeit:

Zu diesem Zweck wurde sowohl ein wasserdispergierbares Granulat obiger Zusammensetzung aus Anhydrat der Verbindung der Formel I der Modifikation I (Formulierung A) als auch ein wasserdispergierbares Granulat aus Anhydrat der Verbindung der Formel I der Modifikation II (Formulierung B) hergestellt.

Anschließend wurde eine 5% ige Suspension der jeweiligen wasserdispergierbaren Granulate in CIPAC D Wasser (Ca⁺⁺/Mg⁺⁺ (4:1) Härte von 342 ppm und pH von 6.0 bis 7.0) hergestellt. Diese Suspension wurde dann mit 1 % Solvesso 200 (Gemisch aromatischer Kohlenwasserstoffe) als repräsentativem Lösungsmittel für lösungsmittelhaltige Flüssigformulierungen (z.B. EC) gemischt. Die entsprechende Spritzbrühe wurde dann über einen bestimmten Zeitraum bei einer bestimmten Temperatur gelagert. Zur Beurteilung der Qualität wurde nach der Lagerung der Rückstand an Feststoff auf einem 75 µm Sieb bestimmt.

### Ergebnis

| **Formulierung** | **Lagerzeit** | **Lagertemperatur** | **Rückstand auf 75 µm Sieb** |
|---|---|---|---|
| A | 24 Stunden | 20°C | 13,0 % w/w |
| A | 24 Stunden | 30°C | 14,3 % w/w |
| A | 7 Tage | 20°C | 16,8 % w/w |
| A | 7 Tage | 30°C | 19,3 % w/w |
| B | 24 Stunden | 20°C | 0,1 % w/w |
| B | 24 Stunden | 40°C | Spuren |
| B | 7 Tage | 20°C | Spuren |
| B | 7 Tage | 40°C | 0,1 % w/w |

## Patentansprüche

1. Monoklines, bei 147 - 148 °C schmelzendes 2-Chlor-N-(4'-chlorbiphenyl-2-yl)-nicotinamid der Formel I

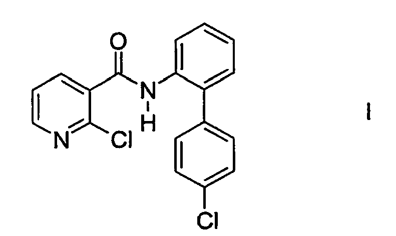

2. Verfahren zu Herstellung der Verbindung I nach Anspruch 1, **dadurch gekennzeichnet, dass** man
a) bei 144 -145 °C schmelzendes 2-Chlor-N-(4'-chlorbiphenyl-2-yl)-nicotinamid der Formel I in einem protisch polaren Lösungsmittel oder einem aromatischen Kohlenwasserstoff löst, und
b) nach Abkühlen aus diesem Lösungsmittel auskristallisiert.

3. Verfahren zur Herstellung der Verbindung I nach Anspruch 2, **dadurch gekennzeichnet, dass** man als protisch polare Lösungsmittel Alkohole, Glykole, Ketone, Ether, Ester, Amide, Dimethylsulfoxid oder deren Gemische verwendet.

4. Verfahren zur Herstellung der Verbindung I nach Anspruch 3, **dadurch gekennzeichnet, dass** man als protisch polare Lösungsmittel Alkohole, Ester oder Ketone verwendet.

5. Verfahren zu Herstellung der Verbindung I nach Anspruch 4, **dadurch gekennzeichnet, dass** man als Alkohol Methanol oder Ethanol verwendet.

6. Verfahren zur Herstellung der Verbindung I nach Anspruch 2, **dadurch gekennzeichnet, dass** man in der Stufe a) die Verbindung der Formel I bei einer Temperatur von 20 °C bis 150 °C löst.

7. Verfahren zur Herstellung der Verbindung I nach Anspruch 2, **dadurch gekennzeichnet, dass** man in der Stufe a) die Verbindung der Formel I bei einer Temperatur von 40 °C bis 115 °C löst.

8. Herbizides Mittel, enthaltend die Verbindung der Formel I gemäß Anspruch 1 und inerte Zusatzstoffe.

9. Herbizides Mittel nach Anspruch 8, **dadurch gekennzeichnet, dass** es 0,1 bis 95 Gew-% der Verbindung der Formel I gemäß Anspruch 1 enthält.

10. Verfahren zur Bekämpfung von Schadpilzen, **dadurch gekennzeichnet, dass** man die Schadpilze, deren Lebensraum oder die von ihnen freizuhaltenden Pflanzen, Flächen, Materialien oder Räume mit einer fungizid wirksamen Menge einer Verbindung der Formel I gemäß Anspruch 1 oder einem die Verbindung der Formel I gemäß Anspruch 1 enthaltenden Mittel gemäß Anspruch 7 behandelt.

## Claims

1. A monoclinic 2-chloro-N-(4'-chlorobiphenyl-2-yl)nicotinamide melting at 147 - 148°C and of the formula I

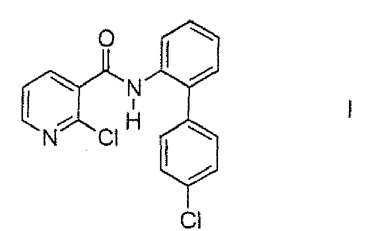

2. A process for the preparation of the compound I according to claim 1, wherein
a) 2-chloro-N-(4'-chlorobiphenyl-2-yl)nicotinamide melting at 144 - 145°C and of the formula I is dissolved in a protic polar solvent or an aromatic hydrocarbon and
b) crystallizes out of the solvent after cooling.

3. The process for the preparation of the compound I according to claim 2, wherein the protic polar solvent used is an alcohol, glycol, ketone, ether, ester or amide or dimethyl sulfoxide or a mixture thereof.

4. The process for the preparation of the compound I according to claim 3, wherein the protic polar solvent used is an alcohol, ester or ketone,

5. The process for the preparation of the compound I according to claim 4, wherein the alcohol used is methanol or ethanol.

6. The process for the preparation of the compound I according to claim 2, wherein the compound of the formula I is dissolved at from 20 to 150°C in stage a).

7. The process for the preparation of the compound I according to claim 2, wherein the compound of the formula I is dissolved at from 40 to 115°C in stage a).

8. A herbicide comprising the compound of the formula I according to claim 1 and inert additives.

9. The herbicide according to claim 8, which comprises from 0.1 to 95% by weight of the compound of the formula I according to claim 1.

10. A method for controlling harmful fungi, wherein the harmful fungi, their habitat or the plants, area or materials or spaces to be kept free from them are treated with a fungicidal amount of a compound of the formula I according to claim 1 or a herbicide according to claim 8 and comprising the compound of the formula I according to claim 1.

## Revendications

1. 2-Chloro-N-(4'-chlorobiphényl-2-yl)-nicotinamide monoclinique, fondant à 147-148°C et répondant à la formule I :

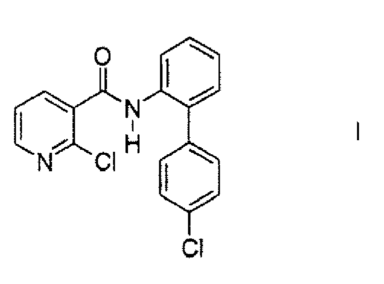

2. Procédé de préparation du composé I suivant la revendication 1, **caractérisé en ce que**
a) on dissout du 2-chloro-N-(4'-chlorobiphényl-2-yl)-nicotinamide de la formule I fondant à 144-145°C dans un solvant polaire protique ou un hydrocarbure aromatique, et
b) on le sépare à l'état cristallin de ce solvant, après refroidissement.

3. Procédé de préparation du composé I suivant la revendication 2, **caractérisé en ce que**, comme solvant polaire protique, on utilise des alcools, des glycols, des cétones, des éthers, des esters, des amides, du diméthylsulfoxyde ou leurs mélanges.

4. Procédé de préparation du composé I suivant la revendication 3, **caractérisé en ce que**, comme solvant polaire protique, on utilise des alcools, des esters ou des cétones.

5. Procédé de préparation du composé I suivant la revendication 4, **caractérisé en ce que**, comme alcool, on utilise du méthanol ou de l'éthanol.

6. Procédé de préparation du composé I suivant la revendication 2, **caractérisé en ce que**, dans l'étape a), on dissout le composé de la formule I à une température de 20°C jusqu'à 150°C.

7. Procédé de préparation du composé I suivant la revendication 2, **caractérisé en ce que**, dans l'étape a), on dissout le composé de la formule I à une température de 40°C jusqu'à 115°C.

8. Produit herbicide contenant le composé de la formule I suivant la revendication 1 et des additifs inertes.

9. Produit herbicide suivant la revendication 8, **caractérisé en ce qu'**il contient 0,1 à 95 % en poids du composé de la formule I suivant la revendication 1.

10. Procédé pour lutter contre des champignons nuisibles, **caractérisé en ce qu'**on traite les champignons nuisibles, leur environnement ou les plantes, surfaces, matériels ou espaces qui doivent en être libérés par une quantité efficace du point de vue fongicide d'un composé de la formule I suivant la revendication 1 ou par un produit suivant la revendication 8 qui contient le composé de la formule I suivant la revendication 1.
